# FASCICULE DE BREVET EUROPEEN

(11) **EP 0 470 317 B1**
(45) Date de publication et mention de la délivrance du brevet: **11.05.1994**
(21) Numéro de dépôt: 90402558.2
(22) Date de dépôt: 17.09.1990
(51) Int. Cl.: A61K 31/52

(54) **Utilisation d'un dérivé de la triméthyl-1,3,7 xanthine pour le traitement des troubles de la mémoire, des troubles intellectuels de la sénescence et de la maladie d'Alzheimer**
Verwendung eines Derivates des 1,3,7-Trimethylxanthins zur Behandlung von Gedächtnisstörungen, intellektuellen Störungen im Alter, und bei der Alzheimerkrankheit
Use of a derivative of 1,3,7-trimethylxanthine for the treatment of memory disorders, senile intellectual disorders and Alzheimer's disease

(30) Priorité: 10.08.1990 FR 9010235
(43) Date de publication de la demande: 12.02.1992
(73) Titulaire: ADIR ET COMPAGNIE, 92415 Courbevoie Cédex (FR)
(72) Inventeur: Kamoun, Annie, F-92200 Neuilly Sur Seine (FR); Mocaer, Elisabeth, F-92200 Neuilly Sur Seine (FR); Regnier, Gilbert, F-92290 Chatenay Malabry (FR); Guillonneau, Claude, F-92140 Clamart (FR); Duhault, Jacques, F-78290 Croissy Sur Seine (FR)
(74) Mandataire: Reverbori, Marcelle

(56) Documents cités:
- EP-A- 0 149 578
- EP-A- 0 260 127
- US-A- 4 755 517

## Description

La présente invention concerne l'utilisation d'un dérivé de la triméthyl-1,3,7 xanthine pour l'obtention de médicaments destinés au traitement des troubles de la mémoire et de la maladie d'Alzheimer.

Plus spécifiquement, l'invention concerne l'utilisation, pour l'obtention de médicaments destinés au traitement des troubles de la mémoire et de la maladie d'Alzheimer, de la triméthyl-1,3,7 [(diéthylaminocarbonyl-4 pipérazino)-3 propyl]-8 xanthine et de ses sels d'addition acide physiologiquement tolérables.

La triméthyl-1,3,7 [(diéthylaminocarbonyl-4 pipérazino)-3 propyl]-8 xanthine et ses sels d'addition acide physiologiquement tolérables sont décrits dans le brevet européens N° EP-A-0.149.578 comme étant utilisables dans le traitement de la migraine et de l'asthénie en raison de leurs propriétés analgésique d'une part et psychotoniques d'autre part, à la dose de 50mg/kg P.O.

La demanderesse a présentement découvert que la triméthyl-1,3,7 [(diéthylaminocarbonyl - 4 pipérazino)-3 propyl]-8 xanthine et ses sels d'addition acide physiologiquement tolérables possèdent des propriétés intéressantes notamment des propriétés facilitatrices de l'aquisition et de la rétention mnésique et des propriétés anti-amnésiques qui permettent de les utiliser pour obtenir des médicaments destinés au traitement des troubles de la mémoire et de la maladie d'Alzheimer, et ceci à des doses spécifiques très inférieures aux doses utilisées antérieurement pour le traitement de la migraine.

La présente invention a également pour objet les compositions pharmaceutiques contenant comme principe actif la triméthyl-1,3,7 [(diéthylaminocarbonyl - 4 pipérazino)-3 propyl]-8 xanthine ou un de ses sels physiologiquement tolérables, mélangé ou associé à un excipient pharmaceutique approprié.

Les compositions pharmaceutiques ainsi obtenues sont présentées avantageusement sous des formes pharmaceutiques convenant pour l'administration par voie orale ou parentérale et notamment intraveineuse, telles que, par exemple les comprimés, dragées, gélules, solutions injectables ou buvables.

La posologie varie selon l'âge et le poids du patient, la voie d'administration, la nature de l'indication thérapeutique et des traitements associés et s'échelonne de 2 à 50 mg de principe actif par prise de 1 à 3 fois/jour.

Les exemples suivants illustrent l'invention et ne la limitent en aucune façon.

Tous les tests qui suivent ont été réalisés en utilisant comme principe actif (en raison notamment de sa bonne solubilité aqueuse) le chlorhydrate de triméthyl-1,3,7 [(diéthylaminocarbonyl-4 pipérazino)-3 propyl]-8 xanthine de formule I :

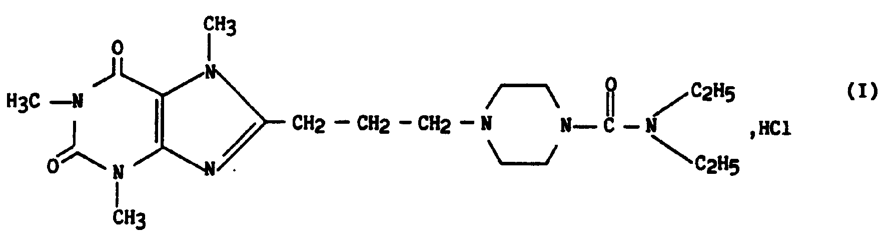

### EXEMPLE 1 :

**EFFET DU PRODUIT I SUR LA VITESSE D'ACQUISITION ET D'APPRENTISSAGE**

Le protocole utilisé décrit par GALEY, DURKIN, SIFAKIS, KEMPF et JAFFARD dans Brain Res., 340, 171-174 (1985) permet :
1) De mesurer **l'alternance spontanée** dont les modifications sont souvent prédictives des effets d'un traitement sur l'apprentissage et la mémoire spatiale.
2) De mesurer **la vitesse d'acquisition** d'une discrimination spatiale simple (droite-gauche) qu'un animal normal maîtrise en une vingtaine d'essais.
3) D'évaluer la **rétention mnésique** de cette discrimination lors d'une épreuve réalisée 24 heures plus tard.

**A)** **Techniques et methodes**
   · Animaux
      Cinquante souris mâles de la lignée BALB/c, vierges de toute expérimentation et âgées de 12 à 14 semaines, ont été utilisées. Elles étaient maintenues en cage individuelle dans une animalerie climatisée (22°C) et pourvue d'un cycle artificiel lumière-obscurité de 12h (8h-20h).
   · Appareil
      C'est un labyrinthe en T construit en plexiglas de couleur grise. Le couloir central et les deux bras d'arrivée mesurent 35 cm de long, 10 cm de large et 25 cm de haut. Le compartiment de départ (10 × 12 cm) est séparé du couloir central par une porte à guillotine. L'entrée de chaque bras est également pourvue d'une porte.
   · Protocole
      Habituation : L'expérience proprement dite est précédée de 2 séances d'habituation de 10 mn (exploration libre ; 1 séance par jour).
      Alternances spontanées et acquisition de la discrimination spatiale : Ces deux épreuves ont lieu le lendemain, sans interruption. Durant les 6 premiers essais, la nourriture est disposée à l'extrémité des deux bras. Lors de chaque essai, l'animal est placé dans le compartiment de départ pendant 30 secondes (intervalle entre essais). La porte donnant accès au couloir central est ouverte et l'animal peut choisir entre les deux bras où il reste enfermé pendant 30 secondes. On constate que l'animal normal alterne ses choix successifs (65 % d'alternance environ). A partir du 7^{ème} essai, la nourriture n'est plus déposée que dans un seul bras, toujours le même. L'épreuve se poursuit jusqu'à ce que l'animal réussisse 5 essais successifs sans erreur (c'est-à-dire aille 5 fois dans le bras renforcé : critère).
      Rétention de la discrimination spatiale : Elle se déroule dans les mêmes conditions que l'acquisition après un délai de 24 heures. L'épreuve se poursuit jusqu'à ce que l'animal réatteigne le critère de 5/5.
   · Groupes
      Les 50 animaux ont été divisés en 5 groupes de 10 sujets :
      - contrôle-contrôle (pas de traitement)
      - solvant-solvant (0,1 ml/10g de NaCl à 7 ‰ dans les mêmes conditions)
      - drogue-drogue (injection de produit I, 30 mn avant chaque épreuve : 0,0625 - 0,25 - 1 mg.kg-¹ par voie intrapéritonéale).
**B)** **Resultats**
   **1) Alternances spontanées**
      Les résultats des 5 groupes sont résumés dans la figure A. La dose minimum (D1 : 0,0625 mg.kg⁻¹) améliore le pourcentage d'alternances (de 59,0 ± 3,1 % à 72,0 ± 5,3 %).
   **2) Acquisition de la discrimination spatiale**
      Les résultats sont résumés dans la figure B. On constate que l'administration du produit I à la dose de 0,0625 mg.kg⁻¹ (D1) réduit de façon hautement significative le nombre d'essais au critère. Le produit I améliore la vitesse d'acquisition de façon dose-dépendante, la dose D1 donnant une vitesse d'acquisition supérieure à la dose D2 et à la dose D3.
   **3) Rétention de la discrimination spatiale**
      Les résultats sont résumés dans la figure C. A la dose de 0,0625 mg.kg⁻¹, le produit réduit très significativement le nombre d'essais au critère ; ce qui n'est pas le cas des doses supérieures. Là encore, il y a un effet facilitateur dose-dépendant, la dose D1 donnant une atteinte du critère significativement plus rapide que D2 et D3.
   **4) Conclusion**
      L'administration i.p. du Produit I, 30 mn avant chaque épreuve améliore l'alternance spontanée, l'acquisition et la rétention, 24 heures plus tard d'une discrimination spatiale (épreuves réalisées dans le labyrinthe en T). Ces facilitations sont observées à la dose la plus faible utilisée dans le protocole (0,0625 mg.kg⁻¹) et non aux deux doses plus élevées (0,025 et 1 mg.kg⁻¹).
   **5) Expression graphique des résultats**

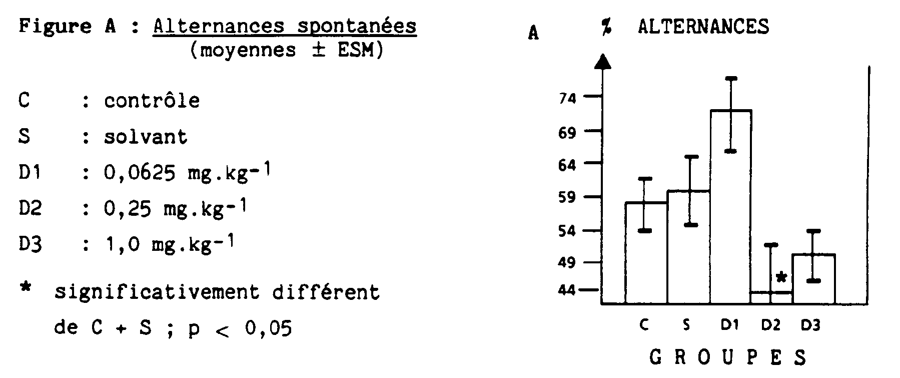

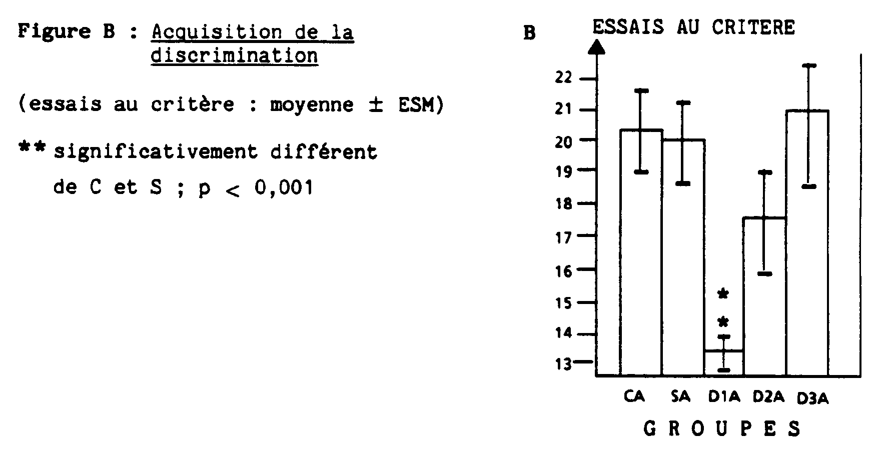

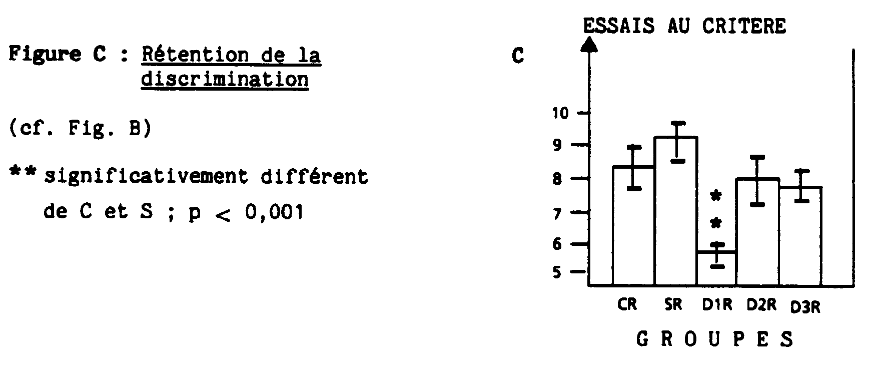

### EXEMPLE 2 :

**EFFET DU PRODUIT I SUR LA CONSERVATION DU PHÉNOMÈNE DE MÉMOIRE** **:**
**1) ETUDE DU CONDITIONNEMENT D'ÉVITEMENT PASSIF CHEZ LE RAT**
   **A)** **Méthodologie** **:**
      Dans ce test, les rats peuvent explorer librement pendant 5 minutes un grand compartiment blanc et un petit compartiment noir. La durée passée dans chaque zone est notée, ainsi que le délai que mettent les rats lâchés dans le compartiment blanc à passer pour la première fois dans le compartiment noir. Chaque rat est ensuite piégé dans le compartiment noir et reçoit dans les pattes un choc électrique impossible à éviter. Une minute plus tard, le rat subit un nouvel essai de 5 minutes, dans lequel il est libre d'aller dans le compartiment blanc ou noir. Le délai de passage dans le compartiment noir et la durée de séjour dans celui-ci sont notés. La fixation mnésique de cette réponse d'évitement passif est ensuite testée chez tous les rats après 48 heures.
      Les doses suivantes du Produit I (exprimé en base) ont été testées : 0,0625, 0,25, 1 et 4 mg/kg. Les rats témoins ont reçu de l'eau distillée ; toutes les injections ont été effectuées par voie intrapéritonéale 30 minutes avant les séances d'acquisition et de test de la fixation mnésique après 48 heures. Dix rats ont été testés pour chaque dose.
   **B)** **Résultats** **:**
      Si l'on considère le délai de passage dans le compartiment noir, on peut voir que les animaux témoins n'ont présenté aucune fixation lors du test réalisé 48 heures plus tard. Par contre, les groupes d'animaux pris dans leur ensemble ont présenté une fixation mnésique.
      Si l'on analyse les données pour tous les groupes, les animaux passent significativement moins de temps du côté noir après 48 heures, par comparaison à la situation avant le choc, mais l'interaction produit x essai est de signification marginale. Si l'on n'analyse que les données des témoins et du groupe 0,0625 mg/kg, l'interaction produit x essai atteint la signification statistique.
**2) MÉMOIRE DE TRAVAIL ET DE RÉFÉRENCE**
   **A)** **Méthodologie** **:**
      Dans ce test, les rats n'ont pas été privés de nourriture, car ils sont récompensés par une friandise sucrée. Avant le début de l'entraînement, les rats ont reçu de telles friandises dans leurs cages pendant cinq jours afin de les y accoutumer.
      Dans chaque essai, les friandises sucrées ont été placées face à 3 trous, sur 9 trous possibles ; la tâche du rat était de ne visiter que les trous avec friandises, de prendre et de manger celle-ci et de ne plus retourner vers le trou à partir du moment où la friandise y a été prise. Ce sont les mêmes trous qui ont reçu l'appât chaque jour, de sorte que les visites aux trous sans appât indiquent des erreurs de la mémoire de référence. De nouvelles visites à un trou dont la friandise a déjà été retirée et consommée indiquent une erreur de la mémoire de travail dans cette étude. Les performances sont analysées par le délai d'achèvement de la tâche à réaliser (retirer et consommer les trois friandises) et par le nombre et la nature des erreurs effectuées. Un essai par jour a été pratiqué. Lorsque les animaux ont satisfait aux critères (réaliser la tâche en moins de 60 secondes, prendre et manger les trois friandises sucrées et ne pas commettre plus d'une erreur), ils ont été laissés au calme dans leurs cages avant de subir un nouveau test 7 jours plus tard. La fixation mnésique a été testée trois semaines plus tard.
      Des rats Lister mâles ont été utilisés ; dix d'entre-eux ont été attribués à chacun des groupes suivants : témoin (eau) ; Produit I (0,0625, 0,25, 1 et 4 mg/kg ; toutes les doses sont exprimées en base). Les injections on été effectuées chaque jour par voie intrapéritonéale, 30 minutes avant le test.
   **B)** Résultats :
      Tous les animaux ont appris à exécuter la tâche. Dans l'ensemble, le Produit I n'a pas significativement modifié le délai nécessaire à l'accomplissement de la tâche.
      Dans l'ensemble, les rats ont mis significativement plus de temps à accomplir la tâche lorsqu'ils ont été testés à nouveau après un intervalle de 7 jours.
      Le Produit I n'a pas eu d'effet global sur le nombre d'erreurs commises.
      Les groupes ont présenté des différences dans le nombre total d'erreurs commises le jour 7. Dans ce cas, le groupe 0,0625 mg/kg a commis significativement **moins** d'erreurs totales que le groupe témoin. Si l'on analyse les différents types d'erreurs commises dans ce test de fixation mnésique, on constate que le groupe 0,0625 mg/kg a commis moins d'erreurs de chaque type, et que la différence est proche de la signification statistique pour les erreurs de la mémoire de référence.
   **3) CONCLUSION**
      Les tâches choisies pour cette étude mesurent des types d'apprentissage différents. Il est manifeste que le Produit I n'exerce aucun effet sur l'accoutumance à long terme, même lorsque l'on utilise le long délai de 48 heures entre les essais.
      Dans la tâche d'apprentissage avec récompense, le Produit I a eu tendance (en particulier à la dose la plus faible) à gêner l'acquisition. Par contre, dans le test de fixation mnésique après une semaine, la plus faible dose (0,0625 mg/kg) a réduit significativment le nombre d'erreurs commises (en particulier les erreurs de la mémoire de référence), ce qui indique une amélioration de la fixation mnésique à long terme de cette tâche. La même dose a amélioré la fixation à long terme (48 heures) de la réponse d'évitement passif. On peut donc conclure que le Produit I améliore la fixation mnésique à long terme des tâches récompensées ou punies.

### EXEMPLE 3 :

**ACTION ANTI-AMNESIQUE DU PRODUIT I** **:**
**1) EFFETS DU PRODUIT I SUR L'AMNÉSIE INDUITE PAR LA SCOPOLAMINE DANS LE TEST D'ÉVITEMENT PASSIF CHEZ LA SOURIS :**
   **A)** **Méthodologie** **:**
      Le test a été réalisé sur des souris mâles de souche N.M.R.I., d'un poids moyen par groupe de 24 à 27 g. Les composés utilisés ont été :
      - le produit I, en solution dans de l'eau distillée ;
      - le piracétam (produit de référence) en solution dans de l'eau distillée ;
      - le bromhydrate de scopolamine en solution dans une solution de Nacl à 0,9 %.

      Tous les essais ont été réalisés à température ambiante (20-21°C) sous lumière artificielle.
      Le test, selon la technique de LENEGRE A. et Coll., Pharmacol. Biochem. Behar., 29, 625-629 (1988), a été réalisé comme suit :
      Une souris est introduite dans le compartiment éclairé (10 x 10 x 29 cm) d'une boîte à deux compartiments. Lorsqu'elle a franchi des quatre pattes le passage donnant accès au compartiment sombre de plus grandes dimensions (19,5 x 16,5 x 29 cm), elle reçoit un léger choc électrique (0,35 mA) jusqu'à ce qu'elle revienne dans le compartiment éclairé duquel elle est immédiatement enlevée (S1). Lorsque la souris est réintroduite 24 heures plus tard dans le système (S2), elle évite de pénétrer dans le compartiment sombre. Le délai de passage dans celui-ci est mesuré, jusqu'à 180 secondes.
      Une injection IP de scopolamine (1 mg/kg) 30 minutes avant S1 diminue la mémoire, ce qui est mesuré par le délai que met la souris à passer dans le compartiment sombre à la séance S2.
      Cette amnésie est diminuée par des agents nootropes tels que le piracétam, cf. SCHINDLER U. et Coll., Drug Dev. Res., 4, 567-576, (1984). 18 animaux sont étudiés dans chaque groupe. L'expérimentation est effectuée en aveugle.
      Le produit I a été étudié aux doses suivantes : 0,25, 1,4 et 16 mg/kg (première expérience) et 0,0625, 0,125, 0,25 et 0,5 mg/kg (seconde expérience), administrées per os 60 minutes avant S1.
      Le piracétam (2048 mg/kg p.o.), administré dans les mêmes conditions expérimentales, a été employé comme composé de référence dans les deux expériences.
   **B)** **Résultats** **:**
      Dans les conditions expérimentales, le produit I à des doses comprises entre 0,0625 et 0,5 mg/kg, a significativement antagonisé l'amnésie induite par la scopolamine. A des doses plus élevées (1,4 et 16 mg/kg), aucun effet significatif n'a été observé. D'après ces résultats, on peut donc conclure que le produit I possède une activité anti-amnésique sur une large plage de doses.
**2) EFFETS DU PRODUIT I SUR L'AMNESIE INDUITE PAR LE DIAZEPAM ET L'ELECTROCHOC DANS LE TEST D'EVITEMENT PASSIF CHEZ LA SOURIS :**
   **A)** **Méthodologie** **:**
      Les conditions opératoires et le test utilisé sont identiques à ceux décrits ci-dessus [cf. 1)-A], mais l'amnésie est induite ici non par la scopolamine mais par :
      - soit le diazépam utilisé en dispersion dans une suspension de gomme arabique à 5 %.
         Dans ce cas :
         Une injection IP de diazépam (1 mg/kg) 30 minutes avant S1 diminue la mémoire, ce qui est mesuré par le délai que met la souris à passer dans le compartiment sombre à la séance S2. Cette amnésie est diminuée par des agents nootropes tels que le piracétam, cf. SCHINDLER U., Drug Dev. Res., 4, 567-576, (1984).
         18 animaux sont étudiés dans chaque groupe. L'expérimentation est effectuée en aveugle.
         Le produit I a été étudié aux doses suivantes : 0,0312, 0,0625, 0,125 et 0,25 mg/kg, administrées per os 60 minutes avant S1.
         Le piracétam (2048 mg/kg p.o.), administré dans les mêmes conditions expérimentales, a été employé comme composé de référence.
      - soit par l'électrochoc.
         Dans ce cas :
         Un électrochoc (courant rectangulaire, 0,4 s, 50 mA, 50 Hz, par l'intermédiaire d'électrodes temporales connectées à un générateur de chocs Ugo Basile) administré immédiatement après S1 diminue la mémoire, ce qui est mesuré par le délai que met la souris à passer dans le compartiment sombre à la séance S2. Cette amnésie est diminuée par des agents nootropes tels que la piracétam, cf. BANFI et Coll., J. Pharmacol. Meth., 8, 255-263, (1982).
         18 animaux sont étudiés dans chaque groupe. L'expérimentation est effectuée en aveugle.
         Le produit I a été étudié aux doses suivantes : 0,0312, 0,0625, 0,125 et 0,25 mg/kg, administrées per os 60 minutes avant S1.
         Le piracétam (2048 mg.kg p.o.), administré, dans les mêmes conditions expérimentales, a été employé comme composé de référence.
   **B)** **Résultats** **:**
      Dans les conditions opératoires ci-dessus, le produit I :
      - a antagonisé de façon dose-dépendante les effets amnésiants du diazépam, avec un antogonisme de 97 % pour la plus forte dose testée (0,25 mg/kg) ;
      - n'a pas antagonisé les effets amnésiants de l'électrochoc, quelle que soit la dose.

      Le piracétam (2048 mg/kg per os), dans les mêmes conditions expérimentales, a antagonisé les effets amnésiants du diazépam de 89 % et ceux de l'électrochoc de 83 %.
**3) EFFETS DE L'ADMINISTRATION UNIQUE ET REITEREE DU PRODUIT I SUR L'AMNESIE INDUITE PAR LA SCOPOLAMINE DANS LE TEST D'EVITEMENT PASSIF CHEZ LA SOURIS :**
   **A)** **Méthodologie** **:**
      Ce test, qui traduit les effets du produit I en traitement aigu d'une part et chronique d'autre part, a été effectué sur des souris mâles de souche N.M.R.I. d'un poids moyen par groupe de 25 à 26 g.
      Les conditions opératoires et le test utilisé sont identiques à ceux précédemment décrits (cf. 1/-A).
      Les doses de produit I étudiées ont été :
      - **En administration unique :** 0,0312 mg/kg, administrées 60 minutes avant l'essai d'apprentissage (S1) le jour A. De J1 à J3, les animaux ont reçu de l'eau distillée deux fois par jour (à 09h00 et 16h00).
      - **En administration réitérée :** 0,0312 et 0,25 mg/kg, administrées deux fois par jour (à 09h00 et 16h00) pendant trois jours et 60 minutes avant S1 le jour 4.

      Le piracétam (2048 mg/kg), en administration unique dans les mêmes conditions expérimentales, a été employé comme composé de référence.
   **B)** **Résultats** **:**
      Dans les conditions expérimentales ci-dessus, le produit I, en administration unique aux deux doses testées, a nettement antagonisé l'amnésie induite par la scopolamine (antagonisme de 87 % à la dose de 0,0312 mg/kg et de 91 % à 0,25 mg/kg).
      L'administration réitérée des mêmes doses sur une période de quatre jours a entraîné un antagonisme plus marqué de l'amnésie induite par la scopolamine, dans les mêmes conditions expérimentales (de 93 % et 106 % respectivement). La différence entre l'administration unique et l'administration réitérée a été significative à la dose de 0,25 mg/kg.
      Selon ces résultats, on peut conclure que l'administration réitérée de produit I :
      - n'induit pas d'épuisement des effets anti-amnésiques observés après administration unique,
      - potentialise ses effets anti-amnésiques.

      Toutefois, comme l'antagonisme de l'amnésie après administration réitérée de la plus faible dose n'a pas été maximal et est resté inférieur à celui obtenu avec le composé de référence (le piracétam), cette potentialisation n'est pas très marquée.
**4) ETUDE DE LA SPECIFICITE DE L'EFFET ANTI-AMNESIQUE DU PRODUIT I :**
   En complément des tests ci-dessus décrits, le produit I a été étudié après administration orale à la souris mâle de souche N.M.R.I. afin d'évaluer ses interactions éventuelles avec les effets de la scopolamine (1 mg/kg IP) sur l'activité locomotrice spontanée et avec les effets du diazépam (1 mg/kg IP) dans le test des quatre plaques (activité anxiolytique).
   La dose employée a été de 0,25 mg/kg, administrée 60 minutes avant les tests (c'est-à-dire 30 minutes avant l'injection IP de scopolamine ou de diazépam). Il s'agit de la plus forte dose de produit I qui a antagonisé l'amnésie induite par la scopolamine ou le diazépam dans le test d'évitement passif (1, 2).
   Les résultats obtenus montrent :
   - l'absence d'antagonisme de l'hypermotilité induite par la scopolamine dans l'activité-mètre ;
   - l'absence d'antagonisme du déblocage, induit par le diazépam, du comportement puni dans le test des quatre plaques.

   Ces résultats confirment que les effets anti-amnésiques du produit I ne sont pas liés à la nature spécifique de ces deux agents inducteurs d'une amnésie, et permettent de conclure à la spécificité de l'action anti-amnésique du produit I à faible dose.

## Revendications

1. Utilisation, pour l'obtention de médicaments destinés au traitement des troubles de la mémoire et de la maladie d'Alzheimer, de la triméthyl-1,3,7 [(diéthylaminocarbonyl-4 pipérazino)-3 propyl]-8 xanthine et de ses sels d'addition acide physiologiquement tolérables.

2. Utilisation, selon la revendication 1, pour l'obtention de médicaments destinés au traitement des troubles de la mémoire et de la maladie d'Alzheimer, du chlorhydrate de triméthyl-1,3,7 [(diéthylaminocarbonyl-4 pipérazino)-3 propyl]-8 xanthine.

## Patentansprüche

1. Verwendung von 1,3,7-Trimethyl-8- [3-(4-diethylaminocarbonyl-piperazino)-propyl]-xanthin und dessen physiologisch verträglichen Säureadditionssalzen zur Herstellung von Arzneimitteln zur Behandlung von Gedächtnisstörungen und der Alzheimerschen Krankheit.

2. Verwendung nach Anspruch 1 von 1,3,7-Trimethyl-8-[3-(4-diethylaminocarbonyl-piperazino)-propyl]-xanthin-hydrochlorid zur Herstellung von Arzneimitteln zur Behandlung von Gedächtnisstörungen und der Alzheimerschen Krankheit.

## Claims

1. Use of 1,3,7-trimethyl-8-[3-(4-diethylaminocarbonylpiperazino)propyl]xanthine and its physiologically tolerable acid addition salts for the preparation of medicaments for the treatment of memory disorders and Alzheimer's disease.

2. Use according to claim 1 of 1,3,7-trimethyl-8-[3-(4-diethylaminocarbonylpiperazino)propyl]xanthine hydrochloride for the preparation of medicaments for the treatment of memory disorders and Alzheimer's disease.
